# EUROPEAN PATENT APPLICATION

(11) **EP 4 728 964 A1**
(43) Date of publication of application: **22.04.2026**
(21) Application number: 23941347.9
(22) Date of filing: 28.11.2023
(51) Int. Cl.: A61B 1/04, A61B 1/06, A61B 1/00, A61B 1/005

(54) **IMAGING MODULAR UNIT AND ENDOSCOPE**

(30) Priority: 16.06.2023 CN 202310726213
(71) Applicant: Guangzhou Red Pine Medical Instrument Co., Ltd., Guangzhou, Guangdong 510000 (CN)
(72) Inventor: YI, Feng, Guangzhou, Guangdong 510000 (CN); LI, Jing, Guangzhou, Guangdong 510000 (CN); YAN, Qianlu, Guangzhou, Guangdong 510000 (CN); ZENG, Lingyue, Guangzhou, Guangdong 510000 (CN)
(74) Representative: Metida
(86) International application number: PCT/CN2023/134536
(87) International publication number: WO 2024/255126

(57) **Abstract**

The present disclosure relates to an imaging assembly and an endoscope. The imaging assembly comprises an image capture module and a second circuit board, wherein the image capture module comprises a camera, a light source component and a first circuit board. The first circuit board comprises a first rigid board, and both the camera and the light source componentare electrically connected to the first rigid board. The second circuit board comprises a second rigid board and a flexible board. One end of the second rigid board is electrically connected to the first rigid board, the second rigid board is arranged at an angle with the first rigid board, and the other end of the second rigid board is electrically connected to the flexible board. The imaging assembly reduces manufacturing process difficulty and increases production efficiency and product yield.

## Description

### FIELD OF TECHNOLOGY

The present disclosure relatesto the field of medical devices, and in particular to an imaging assembly and an endoscope.

### BACKGROUND

Endoscopes are mainly used in surgical operations and routine medical examinations. During clinical use, doctors can insert an insertion portion of the endoscope into a body through natural orifices of the human body or by making small incisions on the body. Then, with the help of other surgical instruments and image processors, internal surgical operations can be performed outside the body. Compared with traditional surgical operations, minimally invasive surgeries using endoscopes have smaller incisions and faster postoperative recovery for patients. Therefore, endoscopes have been widely applied.

An endoscope mainly uses an imaging assembly to illuminate and observe human body's cavities or tissues. To ensure that the insertion portion of the endoscope can smoothly pass through the narrow human body cavities, and to reduce the harm to the patient during the surgery, the size of the imaging assembly is getting smaller and smaller, which makes the manufacturing process of the imaging assembly more difficult to implement. In related technologies, the imaging assembly usually conducts signal output via electronic wires or flexible circuit boards (i.e., FPC). Due to the small size of the imaging assembly and the soft material of the electronic wires or flexible circuit boards, the welding is difficult, the defect rate is high, and the production efficiency is low.

### SUMMARY

Based on the above, it is necessary to provide an imaging assembly for an endoscope and an endoscope that address the issues of how to reduce process difficulty, improve product reliability, and increase product yield.

The imaging assembly for an endoscope comprises an image capture module comprising a camera, a light source component, and a first circuit board, wherein the first circuit board comprises a first rigid board, and both the camera and the light source component are electrically connected to the first rigid board; and a second circuit board comprising a second rigid board and a flexible board, wherein one end of the second rigid board is electrically connected to the first rigid board, and the second rigid board is arranged at an angle to the first rigid board, and the other end of the second rigid board is electrically connected to the flexible board.

The technical solution is further described below:

In one of the embodiments, the first rigid board comprisesa first mounting surface and a second mounting surface disposed opposite to each other; both the camera and the light source component are electrically connected to the first mounting surface; the second rigid board is electrically connected to the second mounting surface and is arranged at an angle to the second mounting surface.

In one of the embodiments, the second rigid board is arranged at a right angle to the second mounting surface.

In one of the embodiments, the second rigid board comprises anend segment and a narrowed segment that are connected to each other; the end segment is electrically connected to the first rigid board; the narrowed segment is electrically connected to the flexible board; a width of the narrowed segment is less than a width of the end segment.

In one of the embodiments, a width of the flexible board is less than or equal to the width of the narrowed segment.

In one of the embodiments, the imaging assembly further comprises a sleeve, wherein the narrowed segment is provided with an electronic component; the sleeve is sleeved over the narrowed segment and covers the electronic component.

In one of the embodiments, the sleeve is a heat-shrink tube that configured to, upon heating, shrink to wrap around the narrowed segment.

In one of the embodiments, the first rigid board comprises a first mounting surface and a second mounting surface disposed opposite to each other; both the camera and the light source component are electrically connected to the first mounting surface; and the end segment is electrically connected to the second mounting surface.

In one of the embodiments, the end segment is provided with a contact point; the end segment is soldered to the second mounting surface via the contact point.

In one of the embodiments, a reinforcing adhesive is applied between the end segment and the second mounting surface.

In one of the embodiments, the imaging assembly further comprises a connector, wherein the connector is electrically connected to an end of the flexible board distal from the second rigid board; the connector is configured to electrically connect to a circuit board within a handle of the endoscope.

In one of the embodiments, the imaging assembly further comprises an electrostatic discharge component for releasing static electricity, wherein the electrostatic discharge component is disposed through the first rigid board.

In one of the embodiments, the imaging assembly further comprises a distal-end housing, wherein the distal-end housing is provided with an accommodating cavity for receiving the camera and the first rigid board; a front end of the distal-end housing is provided with a first mounting hole in communication with the accommodating cavity; the light source component is disposed in the first mounting hole and is electrically connected to the first rigid board via an electrical wire.

In one of the embodiments, the light source component is disposed on the first rigid board; the imaging assembly further comprises a distal-end housing provided with an accommodating cavity for receiving the image capture module; a light-guide component is disposed within the accommodating cavity; one end of the light-guide component abuts against the light source component; the other end of the light-guide component extends to the front end of the distal-end housing.

In one of the embodiments, the light-guide component and the distal-end housing are integrally formed by injection molding.

In one of the embodiments, the camera comprises a lens and an imaging chip; the imaging chip is electrically connected to the first rigid board; the lens is connected to a side of the imaging chip facing away from the first rigid board; the image capture module further comprises a spacer block connected to the first rigid board; the light source component is connected to an end of the spacer block distal from the first rigid board.

In one of the embodiments, a distance from a light-emitting surface of the light source component to the first rigid board is greater than a distance from a junction between the imaging chip and the lens to the first rigid board; the distance from the light-emitting surface to the first rigid board is less than or equal to a distance from a front-end surface of the lens to the first rigid board.

In one of the embodiments, the spacer block comprises an insulating component and a conductive component attached to the insulating component; the conductive component is electrically connected to the light source component and the first rigid board.

In one of the embodiments, conductive components are attached on both sides of the insulating component; the two conductive components are respectively connected to a positive electrode and a negative electrode of the light source component.

According to another aspect, this disclosure also provides an endoscope, comprising an insertion portion; a handle; and the imaging assembly. Wherein, a distal end of the insertion portion is connected to the imaging assembly; a proximal end of the insertion portion is connected to the handle; the second circuit board is configured to extend through the insertion portion; and an end of the flexible board distal from the second rigid board is configured to electrically connect to a circuit board within the handle.

In the imaging assembly, by configuring the first circuit board to include a rigid first rigid board and the second circuit board to include a rigid second rigid board and a flexible board, the rigid second rigid board and the first rigid board do not bend or deform when the second circuit board is soldered to the first circuit board. This facilitates alignment for soldering the second rigid board to the first rigid board, reduces the difficulty of the soldering process, improves production efficiency and product yield. Furthermore, the second rigid board and the first rigid board are arranged at an angle, which helps reduce the size of the imaging assembly. Simultaneously, the rigid second rigid board provides more stable support for the image capture module, enhancing structural stability of the image capture module. During the assembly of the imaging assembly into the insertion portion, the second rigid board can serve as a gripping portion, making it convenient for both manual and automated assembly. Meanwhile, the flexible board can bend and deform, allowing it to better conform to the curvature angles of the insertion portion and the handle. As a result, the flexible board can easily pass through the insertion portion of the endoscope and establish an electrical connection to the circuit board within the handle, ensuring stable signal transmission between the image capture module and the handle.

### BRIEF DESCRIPTION

The accompanying drawings, which form a part of this specification, are included to provide a further understanding of the present disclosure. The illustrative embodiments of the present disclosure and the descriptions thereof are intended to explain the present disclosure and do not constitute an undue limitation of the present disclosure.

To describe the technical solutions in the embodiments of the present disclosure more clearly, the following briefly introduces the accompanying drawings required for describing the embodiments. Obviously, the accompanying drawings in the following description show merely some embodiments of the present disclosure. A person of ordinary skill in the art may derive other drawings from these accompanying drawings without creative efforts.

Furthermore, the drawings are not drawn to a 1:1 scale, and the relative dimensions of various components are depicted schematically in the drawings and may not represent true proportions. In the drawings:
FIG. 1 is an exploded view of an imaging assembly according to an embodiment.
FIG. 2 is a schematic structural diagram of the imaging assembly shown in FIG. 1 with adistal-end housing and alight source component omitted.
FIG. 3 is a front view of the imaging assembly shown in FIG. 2.
FIG. 4 is a schematic structural diagram of a second circuit board according to an embodiment.
FIG. 5 is a schematic diagram showing the cooperation between the second circuit board shown in FIG. 4 and asleeve.
FIG. 6 is a schematic structural diagram of a rear end of a flexible board according to an embodiment.
FIG. 7 is an exploded view of an imaging assembly according to another embodiment.
FIG. 8 is a schematic structural diagram of an image capture module of the imaging assembly shown in FIG. 7.
FIG. 9 is a schematic structural diagram of a spacer block of the imaging assembly shown in FIG. 7.
FIG. 10 is an exploded view of an imaging assembly according to yet another embodiment.
FIG. 11 is a schematic structural diagram of an image capture module of the imaging assembly shown in FIG. 10.
FIG. 12 is a cross-sectional view of the distal-end housing of the imaging assembly shown in FIG. 10.

### Reference Numeral Description:

11: first rigid board; 111: first mounting surface; 112: second mounting surface; 20: second circuit board; 21: second rigid board; 211: end segment; 212: narrowed segment; 213: electronic component; 22: flexible board; 23: sleeve; 24: connector; 30: camera; 31: lens; 32: imaging chip; 41: light source component; 42: spacer block; 421: conductive component; 422: insulating component; 51: electrostatic discharge component; 60: distal-end housing; 61: mounting hole; 62: imaging aperture; 63: light-guide component.

### DETAILED DESCRIPTION

In order to make the above objectives, features, and advantages of the present disclosure more apparent and readily understandable, a detailed description of specific embodiments of the present disclosure will now be provided below with reference to the accompanying drawings. Numerous specific details are set forth in the following description to facilitate a thorough understanding of the present disclosure. However, it should be understood that the present disclosure may be embodied in many other forms different from those described herein. Those skilled in the art can make similar modifications without departing from the spirit of the present disclosure. Therefore, the present disclosure is not limited by the specific embodiments disclosed below.

In the description of the present disclosure, it is to be understood that terms such as "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential", and the like, should be construed to refer to the orientation or positional relationship as then described or as shown in the accompanying drawings. These terms are used merely for the purpose of facilitating the description of the present disclosure and simplifying the description, and do not individually or in combination indicate or imply that the referred apparatus or element must have a specific orientation or be constructed and operated in a specific orientation. Therefore, these terms are not to be construed as limiting the present disclosure.

Furthermore, it is to be understood that terms such as "first", "second," and the like are used for descriptive purposes only and are not to be construed as indicating or implying relative importance or implicitly specifying the quantity of the indicated technical features. Thus, features defined by "first", "second", and the like may explicitly or implicitly include at least one of the features. In the description of the present disclosure, the term "a plurality of" means at least two, such as two, three, etc., unless explicitly defined otherwise.

In the present disclosure, unless expressly specified and defined otherwise, terms such as "mounted", "connected", "coupled", "fixed", and the like shall be construed broadly. For example, a connection may be a fixed connection, a detachable connection, or an integral connection; it may be a mechanical connection, an electrical connection, or a communicative connection; it may be a direct connection, or an indirect connection through an intermediary medium; it may be a communication between the interiors of two elements or an interactional relationship between two elements, unless explicitly defined otherwise. A person of ordinary skill in the art may understand the specific meanings of the foregoing terms in the present disclosure according to specific situations.

In the present disclosure, unless expressly specified and defined otherwise, descriptions indicating that a first feature is "on", "above", "below", or similar prepositions relative to a second feature are to be construed broadly. The description may mean that the first and second features are in direct contact, or that the first and second features are not in direct contact but are indirectly connected via an intermediate medium. Furthermore, terms such as "on", "over", "above", and the like may indicate that the first feature is directly above, diagonally above, or merely at a higher level than the second feature. Similarly, terms such as "under", "below", "beneath", and the like may indicate that the first feature is directly below, diagonally below, or merely at a lower level than the second feature.

It should be noted that if an element is referred to as being "fixed to" or "disposed on" another element, it maybe directly on the other element, or intervening elements may be present. If an element is referred to as being "connected" to another element, it maybe directly connected to the other element, or intervening elements may be present simultaneously. Where applicable, the terms "vertical", "horizontal", "upper", "lower", "left", "right", and similar expressions used in this application are for illustrative purposes only and do not denote the only mode of implementation.

An embodiment of the present disclosure provides an endoscope, configured to acquire image information of a target lumen or tissue. Specifically, the endoscope comprises an insertion portion, a handle, and an imaging assembly. A distal end of the insertion portion is connected to the imaging assembly, and a proximal end of the insertion portion is connected to the handle. The imaging assembly is configured to illuminate the target lumen or tissue and capture image information. The imaging assembly is electrically connected to a circuit board within the handle.

Specifically, in a conventional endoscope, an imaging assembly typically comprises a light source component for illumination and a camera for acquiring image information. A circuit board within a handle is configured to electrically connect with the imaging assembly to coordinate operation of the imaging assembly. An insertion portion is usually relatively long and may require a certain degree of bendability. Simultaneously, an angle is often formed between the handle and the insertion portion. To ensure the bendability of the insertion portion and to facilitate wiring between the imaging assembly and the circuit board within the handle, the imaging assembly is typically electrically connected to the circuit board within the handle via soft electronic wires or a flexible circuit board. However, due to a flexible nature of the electronic wires or the flexible circuit board and a generally small size of the imaging assembly, alignment is difficult to control during soldering, leading to soldering difficulties, a high defect rate, and a reduction of production efficiency. Furthermore, the soft electronic wires or the flexible circuit board cannot adequately support the imaging assembly, resulting in an unstable overall structure of the imaging assembly, poor reliability, and inconvenience of assembly.

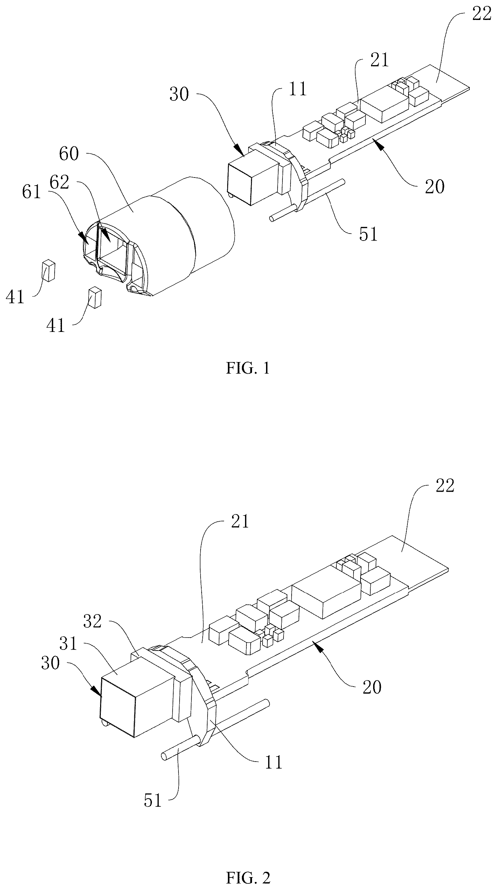

Based on the above, an embodiment of the present disclosure further provides an imaging assembly. Specifically, referring to FIG. 1 to FIG. 3, an imaging assembly comprises an image capture module and a second circuit board 20. The image capture module comprises a camera 30, a light source component 41, and a first circuit board. The first circuit board comprises a first rigid board 11. Both the camera 30 and the light source component 41 are electrically connected to the first rigid board 11, wherein the camera 30 is configured to acquire image information of the target lumen or tissue, and the light source component 41 is configured for illumination. The second circuit board 20 comprises a second rigid board 21 and a flexible board 22. One end of the second rigid board 21 is electrically connected to the first rigid board 11, and the second rigid board 21 is arranged at an angle to the first rigid board 11. The other end of the second rigid board 21 is electrically connected to the flexible board 22. Furthermore, the image capture module is configured to be disposed at a distal end of the insertion portion. The second circuit board 20 is configured to extend through the insertion portion, wherein an end of the flexible board 22 distal from the second rigid board 21 is configured to electrically connect to a circuit board within a handle. Preferably, in the present embodiment, the first rigid board 11 and the second rigid board 21 are both rigid PCB boards, and the flexible board 22 is a flexible FPC board.

Specifically, during manufacturing, the camera 30 and the light source component 41 are first assembled onto the first rigid board 11 to form an integrated image capture module. Then, one end of the second rigid board 21 of the second circuit board 20 is assembled to the first rigid board 11, thereby forming an integrated imaging assembly. Compared to a traditional imaging assembly that routes signals via electronic wires or a flexible circuit board, the imaging assembly of the present disclosure configures the first circuit board to comprise the first rigid board 11 which is rigid, and configures the second circuit board 20 to comprise the second rigid board 21 which is rigid and the flexible board 22 which is flexible. When the second circuit board 20 being soldered to the first rigid board 11, the second rigid board 21 and the first rigid board 11 do not bend or deform due to the rigid nature of the second rigid board 21 and the first rigid board 11, thereby facilitating alignment when soldering the second rigid board 21 to the first rigid board 11, reducing the difficulty of the soldering process and increasing production efficiency and product yield. Furthermore, the second rigid board 21 is arranged at an angle to the first rigid board 11, which is advantageous for reducing the size of the imaging assembly. Simultaneously, the second rigid board 21 that is rigid can also provide more stable support for the image capture module, improving the structural stability of the image capture module. When assembling the imaging assembly into the insertion portion, the second rigid board 21 can serve as a grippingportion, facilitating both manual assembly and automated assembly. Meanwhile, the flexible board 22 which is flexible can bend and deform, allowing it to better conform to the curved angles of the insertion portion and the handle. Consequently, the flexible board 22 can easily pass through the insertion portion of the endoscope and establish an electrical connection to the circuit board within the handle, ensuring stable signal transmission between the image capture module and the handle.

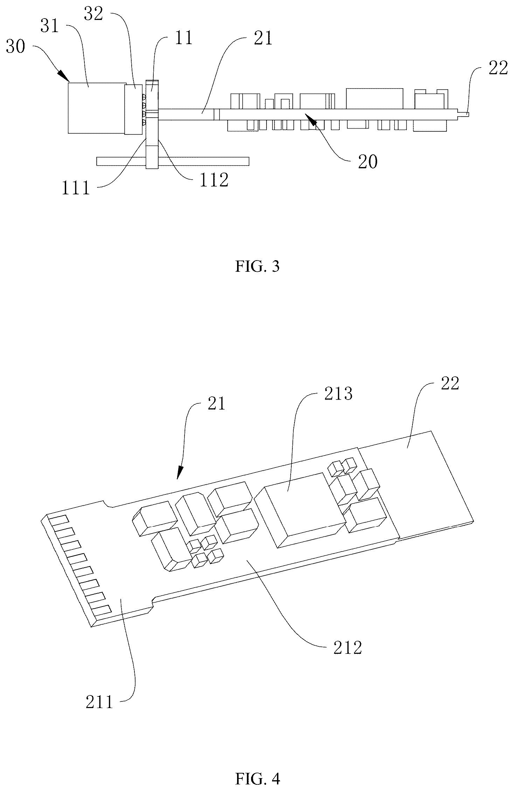

Referring to FIG.3, optionally, in an embodiment, the first rigid board 11 comprises a first mounting surface 111 and a second mounting surface 112 disposed opposite to each other. Both the camera 30 and the light source component 41 are electrically connected to the first mounting surface 111. The second rigid board 21 is electrically connected to the second mounting surface 112, and the second rigid board 21 is arranged at an angle to the second mounting surface 112. Preferably, in the present embodiment, the second rigid board 21 is arranged at a right angle to the second mounting surface 112, thereby causing the second rigid board 21 and the first rigid board 11 to form a structurally stable and reliable T-shaped configuration, which saves space and facilitates wiring.

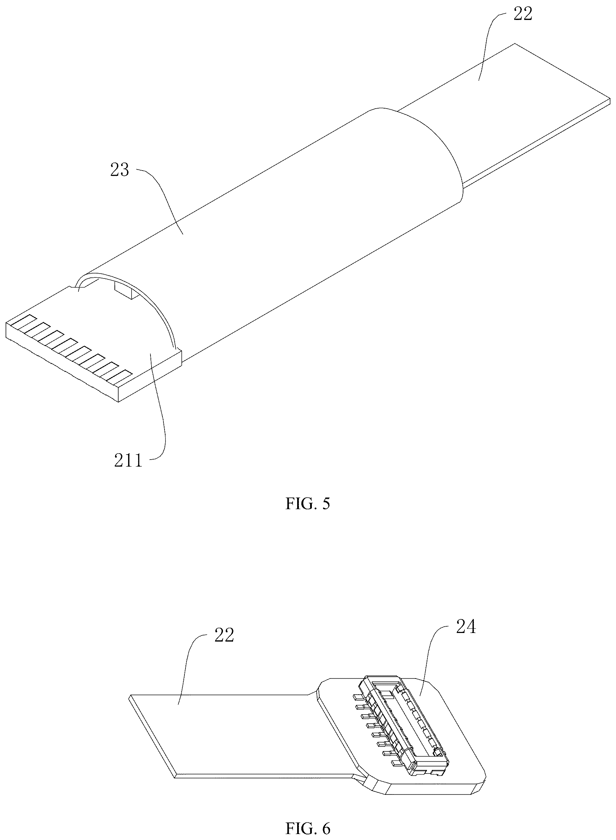

Referring to FIG.5, optionally, in an embodiment, the imaging assembly further comprises a sleeve 23. The sleeve 23 is sleeved over the second circuit board 20, thereby protecting the second circuit board 20 against electrostatic discharge, water and dust.

Further, with reference to FIG. 4, the second rigid board 21 comprises anend segment 211 and a narrowed segment 212 that are connected to each other. The end segment 211 is electrically connected to the first rigid board 11. The narrowed segment 212 is connected to the flexible board 22, and a width of the narrowed segment 212 is less than a width of the end segment 211. A width of the flexible board 22 is less than or equal to the width of the narrowed segment 212. Electronic components 213, such as resistors and capacitors, are disposed on the narrowed segment 212. The sleeve 23 is sleeved over the narrowed segment 212 and covers the electronic components 213, thereby providing protection for the electronic components 213 on the narrowed segment 212. Specifically, to allow the insertion portion to pass smoothly through narrow lumens of the human body and avoid damaging human tissue, the insertion portion is typically slender. Correspondingly, a lumen of the insertion portion is also relatively small. Furthermore, in addition to accommodating the second circuit board 20, the lumen of the insertion portion must reserve space for an instrument channel through which surgical tools pass. Therefore, the size of the second circuit board 20 needs to be minimized as much as possible. By configuring the narrowed segment 212 and the flexible board 22 both to have a small width, and by sleeving the sleeve 23 over the narrowed segment 212, protection for the electronic components 213 on the second circuit board 20 is achieved, while simultaneously avoiding an overall size of a combination of the second circuit board 20 and the sleeve 23 from becoming too large.

Preferably, with reference to FIG.5, the sleeve 23 is a heat-shrink tube. The heat-shrink tube is configured to, upon heating, shrink to wrap around the narrowed segment 212, which provides convenient processing and results in a smaller size. Specifically, the end segment 211 is electrically connected to the second mounting surface 112. The end segment 211 is provided with contact points and is soldered to the second mounting surface 112 via these contact points, thereby achieving a reliable weld. Additionally, a reinforcing adhesive is applied between the end segment 211 and the second mounting surface 112 to enhance the overall structural stability.

Referring to FIG.6, in an embodiment, the imaging assembly further comprises a connector 24. The connector 24 is electrically connected to an end of the flexible board 22 distal from the second rigid board 21. The connector 24 is configured to electrically connect to a circuit board within the handle of the endoscope. In this manner, during assembly, it is only necessary to plug the connector 24 into the circuit board within the handle to establish an electrical connection between the flexible board 22 and the circuit board. This eliminates the need for a soldering process and improves assembly efficiency.

Referring to FIG.2, the imaging assembly further comprises an electrostatic discharge component 51 for releasing static electricity. The electrostatic discharge component 51 is disposed through the first rigid board 11. The electrostatic discharge component 51 is configured to conduct ambient static electricity away, thereby eliminating static electricity on the first rigid board 11 to provide electrostatic protection for components such as the camera 30 and the light source component 41 on the first rigid board 11, and to prevent the camera 30 and the light source component 41 from being affected by electromagnetic interference. Preferably, a through hole is provided in the first rigid board 11, and the electrostatic discharge component 51 is disposed through the through hole. Furthermore, a quantity of the electrostatic discharge component 51 is at least two. The at least two electrostatic discharge components 51 are spaced apart and disposed through the first rigid board 11, thereby improving the effect of electrostatic protection and electromagnetic interference suppression. In some preferred embodiments, the electrostatic discharge component 51 is a metal pin.

Referring to FIG.2, in an embodiment, the camera 30 comprises a lens 31 and an imaging chip 32. The imaging chip 32 is electrically connected to the first rigid board 11. The lens 31 is connected to a side of the imaging chip 32 facing away from the first rigid board 11. Preferably, the imaging chip 32 is electrically connected to the first mounting surface 111 of the first rigid board 11 via a conductive adhesive. The lens 31 is then bonded and packaged to the imaging chip 32 using the conductive adhesive.

Referring to FIG.1, the imaging assembly further comprises a distal-end housing 60, configured to provide waterproof and dustproof protection for the image capture module. Specifically, the distal-end housing 60 is provided with an accommodating cavity for receiving the camera 30 and the first rigid board 11. A front end of the distal-end housing 60 is provided with an imaging aperture 62 in communication with the accommodating cavity, and the camera 30 is disposed in the imaging aperture 62.Optionally, in an embodiment, during manufacturing, the distal-end housing 60 may be first formed by injection molding. Subsequently, the image capture module is placed into the accommodating cavity of the distal-end housing 60. Finally, a potting compound is introduced into the accommodating cavity, thereby fixedly connecting the distal-end housing 60 and the image capture module. In another embodiment, the image capture module may first be fixed in a mold for the distal-end housing 60. A potting compound is then introduced into the mold, followed by curing and demolding, thereby integrally forming the distal-end housing 60 around the image capture module.

Referring to FIG.1, optionally, in an embodiment, the front end of the distal-end housing 60 is further provided with a first mounting hole 61 in communication with the accommodating cavity. The light source component 41 is disposed in the mounting hole 61 and is electrically connected to the first rigid board 11 via electrical wires. In this way, the light source component 41 maybe fixed at the front end of the distal-end housing 60, avoiding obstruction of light emitted by the light source component 41 and resulting in more uniform illumination of the target inspection area. Preferably, a quantity of the light source component 41 is at least two. Correspondingly, the front end of the distal-end housing 60 is provided with at least two mounting holes 61. The light source components 41 are respectively disposed in the mounting holes 61 in a one-to-one correspondence. Furthermore, the light source component 41 may be fixed in the mounting hole 61 via an adhesive. During installation, the camera 30 and the first circuit board 11 are first placed into the accommodating cavity from a rear end of the distal-end housing 60. Then, the light source component 41, with the electrical wire connected, is placed into the mounting hole 61 from the front end of the distal-end housing 60 and fixed by spot gluing. Finally, the electrical wire is connected to the first circuit board 11 to complete the installation of the light source component 41. Preferably, in the present embodiment, the light source component 41 maybe an LED light.

It is worth noting that, in other embodiments, the light source component 41 may alternatively be mounted on the first rigid board 11. In this configuration, heat generated by the light source component 41 maybe transferred through the first rigid board 11 to the second circuit board 20. This avoids direct transfer of the heat from the light source component 41 to a patient via the distal-end housing 60 and causing thermal injury. Simultaneously, it prevents heat from being concentrated at the front end of the distal-end housing 60. On one hand, this arrangement meets temperature limits stipulated in relevant domestic and international standards for medical devices. On the other hand, the camera 30 is a heat-sensitive component, and overheating maycause noise andunstable imaging. Therefore, by mounting the light source component 41 on the first rigid board 11, the heat generated by the light source component 41 is caused to be transferred rearward through the first rigid board 11 and the second circuit board 20, ensuring stable imaging of the imaging assembly.

Furthermore, mounting the light source component 41 on the first rigid board 11 requires that a suitable relative position is maintained between a light-emitting surface of the light source component 41 and a front-end surface of the lens 31. Specifically, if the light-emitting surface of the light source component 41 is higher than the front-end surface of the lens 31, overexposure of the camera 30 is likely to occur. If the light-emitting surface of the light source component 41 is significantly lower than the front-end surface of the lens 31, then a portion of the light from the light source component 41 may be blocked by a lateral side of the camera 30, resulting in non-uniform illumination of the target inspection area. This, in turn, leads to uneven brightness and color in the captured image, which hinders accurate and rapid diagnosis of a lesion and even potentially leading tomisdiagnosis.

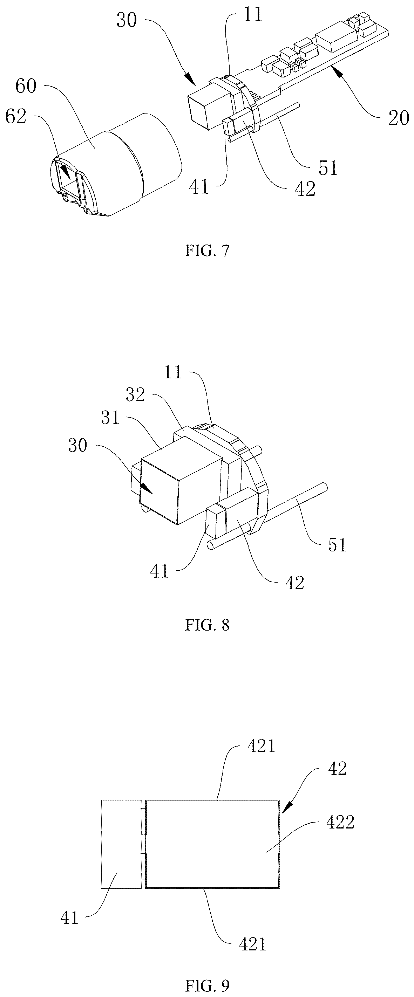

Based on this, with reference to FIGS.7 and 8, in an embodiment, the image capture module further comprises a spacer block 42. The spacer block 42 is connected to the first rigid board 11. The light source component 41 is connected to an end of the spacer block 42 distal from the first rigid board 11, thereby ensuring that a suitable relative position is maintained between the light-emitting surface of the light source component 41 and the front-end surface of the lens 31. Preferably, a distance from the light-emitting surface of the light source component 41 to the first rigid board 11 is greater than a distance from a junction between the imaging chip 32 and the lens 31 to the first rigid board 11. Furthermore, the distance from the light-emitting surface of the light source component 41 to the first rigid board 11 is less than or equal to a distance from the front-end surface of the lens 31 to the first rigid board 11. In this configuration, by elevating the light source component 41 using the spacer block 42, the light-emitting surface of the light source component 41 is positionedto be higher than the junction between the imaging chip 32 and the lens 31. This prevents light emitted from the light source component 41 from entering the junction between the imaging chip 32 and the lens 31 and affecting a photosensitive surface of the imaging chip 32. Moreover, by configuring the light-emitting surface of the light source component 41 to be lower than or flush with the front-end surface of the lens 31, overexposure of the camera 30 is avoided. Simultaneously, blocking of a portion of the light from the light source component 41 by a lateral side of the camera 30 is prevented, resulting in more uniform illumination of the target inspection area and improved image quality. Concurrently, heat from the light source component 41 maybe transferred through the spacer block 42 to the first rigid board 11, and then to the second circuit board 20. This prevents heat concentration at the front end of the distal-end housing 60, thereby avoiding thermal injury to a patient and also avoiding adverse effects on imaging performance of the camera 30.

Furthermore, with reference to FIG.9, the spacer block 42 comprises an insulating component 422 and a conductive component 421 attached to the insulating component 422. The conductive component 421 is electrically connected to the light source component 41 and the first rigid board 11, thereby establishing an effective electrical connection. Specifically, conductive component 421 are attached on both sides of the insulating component 422. The two conductive components 421 are respectively connected to a positive electrode and a negative electrode of the light source component 41. The two conductive components 421 are isolated from each other by the insulating component 422, thereby preventing a short circuit of the light source component 41. Preferably, in the present embodiment, the light source component 41 maybe an LED light.

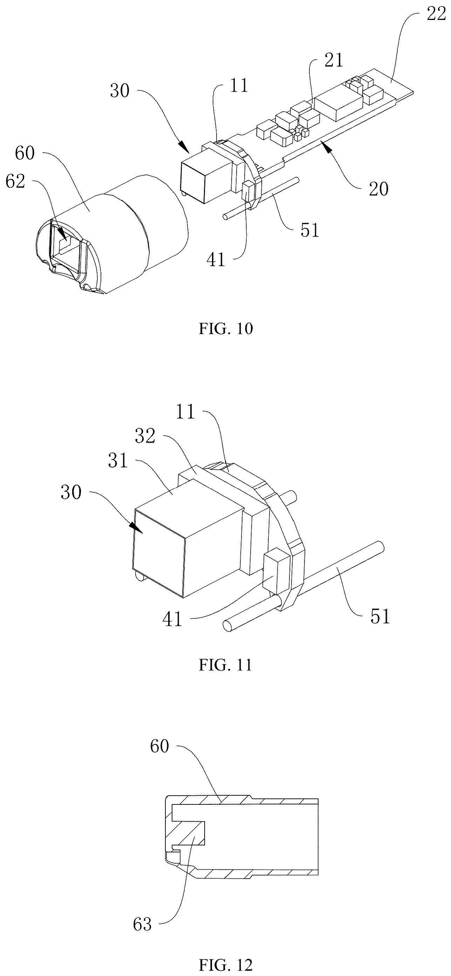

Referring to FIGS. 10 to 12, in yet another embodiment, the light source component 41 is disposed on the first rigid board 11. The accommodating cavity of the distal-end housing 60 is provided with a light-guide component 63. One end of the light-guide component 63 abuts against the light source component 41. The other end of the light-guide component 63 extends to the front end of the distal-end housing 60. In this configuration, light from the light source component 41 is concentrated and transmitted to the front end of the distal-end housing 60 through the light-guide component 63, reducing energy loss of the light emitted by the light source component 41. Consequently, there is no need to be concerned about the light from the light source component 41 being blocked by the camera 30, and scattering of the light from the light source component 41 into the junction between the imaging chip 32 and the lens 31 is also reduced. Optionally, the light-guide component 63 and the distal-end housing 60 are integrally formed by injection molding, resulting in high structural strength. Preferably, in this present embodiment, the light source component 41 maybe an LED light. Additionally, a light-shielding layer or a light-shielding material may be applied to cover the junction between the imaging chip 32 and the lens 31, thereby further reducing the amount of light from the light source component 41 that enters the junctionbetween the imaging chip 32 and the lens 31. Furthermore, heat from the light source component 41 can also be transferred rearward through the first rigid board 11 and the second circuit board 20, ensuring stable imaging of the imaging assembly.

The various technical features described in the aforementioned embodiments can be arbitrarily combined. For the sake of conciseness, not all possible combinations of the technical features in the aforementioned embodiments have been described. However, any combination of these technical features shall be considered as falling within the scope recorded in this specification, provided that no contradiction exists.

The embodiments described above merely represent several implementation modes of the present application. The description is relatively specific and detailed, but it should not be construed as limiting the scope of the present application. It should be noted that a person skilled in the art can make numerous modifications and improvements without departing from the concept of the present application, and all of these fall within the protection scope of the present application.

## Claims

1. An imaging assembly for an endoscope, comprising:
an image capture module comprising a camera, a light source component and a first circuit board, wherein the first circuit board comprises a first rigid board, and both the camera and the light source component are electrically connected to the first rigid board; and
a second circuit board comprising a second rigid board and a flexible board, wherein one end of the second rigid board is electrically connected to the first rigid board, and the second rigid board is arranged at an angle to the first rigid board, and the other end of the second rigid board is electrically connected to the flexible board.

2. The imaging assembly of claim 1, wherein the first rigid board comprises a first mounting surface and a second mounting surface disposed opposite to each other; both the camera and the light source component are electrically connected to the first mounting surface; the second rigid board is electrically connected to the second mounting surface and is arranged at an angle to the second mounting surface.

3. The imaging assembly of claim 2, wherein the second rigid board is arranged at a right angle to the second mounting surface.

4. The imaging assemblyof claim 1, wherein the second rigid board comprises anend segment and a narrowed segment that are connected to each other; the end segment is electrically connected to the first rigid board; the narrowed segment is electrically connected to the flexible board; a width of the narrowed segment is less than a width of the end segment.

5. The imaging assembly of claim 4, wherein a width of the flexible board is less than or equal to the width of the narrowed segment.

6. The imaging assembly of claim 4, further comprising a sleeve, wherein the narrowed segment is provided withan electronic component; the sleeve is sleeved over the narrowed segment and covers the electronic component.

7. The imaging assembly of claim 6, wherein the sleeve is a heat-shrink tube configured to, upon heating, shrink to wrap around the narrowed segment.

8. The imaging assembly of claim 4, wherein the first rigid board comprisesa first mounting surface and a second mounting surface disposed opposite to each other; both the camera and the light source component are electrically connected to the first mounting surface; and the end segment is electrically connected to the second mounting surface.

9. The imaging assembly of claim 8, wherein the end segment is provided with a contact point; the end segment is soldered to the second mounting surface via the contact point.

10. The imaging assembly of claim 9, wherein a reinforcing adhesive is applied between the end segment and the second mounting surface.

11. The imaging assembly of claim 1, further comprising a connector, wherein the connector is electrically connected to an end of the flexible board distal from the second rigid board; the connector is configured to electrically connect to a circuit board within a handle of the endoscope.

12. The imaging assembly of claim 1, further comprising an electrostatic discharge component for releasing static electricity, wherein the electrostatic discharge component is disposed through the first rigid board.

13. The imaging assembly of claim 1, further comprising a distal-end housing, wherein the distal-end housing is provided with an accommodating cavity for receiving the camera and the first rigid board; a front end of the distal-end housing is provided with a first mounting hole in communication with the accommodating cavity; the light source component is disposed in the first mounting hole and is electrically connected to the first rigid board via an electrical wire.

14. The imaging assembly of claim 1, wherein the light source component is disposed on the first rigid board; the imaging assembly further comprises a distal-end housing provided with an accommodating cavity for receiving the image capture module; a light-guide component is disposed within the accommodating cavity; one end of the light-guide component abuts against the light source component; the other end of the light-guide component extends to the front end of the distal-end housing.

15. The imaging assembly of claim 14, wherein the light-guide component and the distal-end housing are integrally formed by injection molding.

16. The imaging assembly of claim 1, wherein the camera comprises a lens and an imaging chip; the imaging chip is electrically connected to the first rigid board; the lens is connected to a side of the imaging chip facing away from the first rigid board; the image capture module further comprises a spacer block connected to the first rigid board; the light source component is connected to an end of the spacer block distal from the first rigid board.

17. The imaging assembly of claim 16, whereina distance from a light-emitting surface of the light source component to the first rigid board is greater than a distance from a junction between the imaging chip and the lens to the first rigid board; the distance from the light-emitting surface to the first rigid board is less than or equal to a distance from a front-end surface of the lens to the first rigid board.

18. The imaging assembly of claim 16, wherein the spacer block comprises an insulating component and a conductive component attached to the insulating component; the conductive component is electrically connected to the light source component and the first rigid board.

19. The imaging assembly of claim 18, wherein conductive components are attached on both sides of the insulating component; the two conductive components are respectively connected to a positive electrode and a negative electrode of the light source component.

20. An endoscope, comprising: an insertion portion; a handle; and the imaging assembly ofanyone of claims 1 to 19, whereina distal end of the insertion portion is connected to the imaging assembly; a proximal end of the insertion portion is connected to the handle; the second circuit board is configured to extend through the insertion portion; and an end of the flexible board distal from the second rigid board is configured to electrically connect to a circuit board within the handle.
